(19) Europäisches Patentamt
European Patent Office
Office européen des brevets

(11) **EP 4 224 236 B1**

(12) # EUROPEAN PATENT SPECIFICATION

(45) Date of publication and mention
of the grant of the patent:
**20.11.2024 Bulletin 2024/47**

(21) Application number: **21875025.5**

(22) Date of filing: **30.08.2021**

(51) International Patent Classification (IPC):
**G02B 23/24** *(2006.01)* **A61B 1/005** *(2006.01)*
**A61M 25/00** *(2006.01)*

(52) Cooperative Patent Classification (CPC):
**A61B 1/0011; A61B 1/00078; B32B 1/08;
B32B 7/12; B32B 15/08; B32B 15/088;
B32B 15/09; B32B 15/095; B32B 15/18;
B32B 27/12; B32B 27/34; B32B 27/36;
B32B 27/40; G02B 23/24;** A61 1/0055; (Cont.)

(86) International application number:
**PCT/JP2021/031740**

(87) International publication number:
**WO 2022/070722 (07.04.2022 Gazette 2022/14)**

(54) **FLEXIBLE TUBE FOR ENDOSCOPE, ENDOSCOPE-TYPE MEDICAL DEVICE, AND METHODS FOR PRODUCING THESE**

FLEXIBLER SCHLAUCH FÜR EIN ENDOSKOP, ENDOSKOPARTIGE MEDIZINISCHE VORRICHTUNG UND VERFAHREN ZU DEREN HERSTELLUNG

TUBE FLEXIBLE POUR ENDOSCOPE, DISPOSITIF MÉDICAL DE TYPE ENDOSCOPE, ET LEURS PROCÉDÉS DE PRODUCTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.09.2020 JP 2020163598**

(43) Date of publication of application:
**09.08.2023 Bulletin 2023/32**

(73) Proprietor: **FUJIFILM Corporation
Tokyo 106-8620 (JP)**

(72) Inventors:
• **HORITA Kazuma
  Ashigarakami-gun, Kanagawa 258-8577 (JP)**
• **NAKAI Yoshihiro
  Ashigarakami-gun, Kanagawa 258-8577 (JP)**
• **ABE Shinya
  Ashigarakami-gun, Kanagawa 258-8577 (JP)**

(74) Representative: **HGF
HGF Limited
1 City Walk
Leeds LS11 9DX (GB)**

(56) References cited:
**EP-A1- 3 653 104       WO-A1-2016/052208
WO-A1-2019/013243    WO-A1-2019/017065
WO-A1-2019/017065    JP-A- 2006 081 662
JP-A- 2006 081 662     JP-A- 2011 212 338
JP-A- 2012 200 353     JP-A- H0 975 299
JP-A- H01 232 923      JP-A- H01 232 923
JP-B1- 6 450 892        US-A- 5 788 714**

**(Cont. next page)**

(52) Cooperative Patent Classification (CPC): (Cont.)
A61M 25/0009; A61M 25/0045; A61M 25/005;
B32B 2255/06; B32B 2274/00; B32B 2307/546;
B32B 2535/00; B32B 2597/00

**Description**

**BACKGROUND OF THE INVENTION**

1. Field of the Invention

The present invention relates to a flexible tube for an endoscope, an endoscopic medical device, and methods for producing the same.

2. Description of the Related Art

Endoscopes are medical devices for examining the inside of the body cavity, the inside of the digestive tract, the esophagus, or the like of a patient. Since endoscopes are inserted and used in the body, it is desirable to provide endoscopes that do not damage organs or cause pain or discomfort to a patient. In view of such a requirement, a flexible-tube base having a spiral tube formed by spirally winding a soft, bendable metal strip and a tubular mesh member that covers the spiral tube is employed for a flexible tube (flexible tube for an endoscope) that forms an insertion section (structural section to be inserted into the body cavity) of an endoscope. Furthermore, the periphery of the flexible-tube base is covered with a flexible resin or elastomer, and the resin or elastomer cover layer is covered with a topcoat layer as required so that the flexible tube does not cause stimulation or damage to the inner surface of, for example, the esophagus, digestive tract, or body cavity.

The flexible tube is required to have high elasticity in order to move smoothly in the body. By increasing elasticity of the flexible tube, the flexible tube that has passed through a bent portion in the body easily returns to a straight shape, and the burden on the subject during a test can be further reduced. For example, WO2019/013243A discloses, as a technology that meets this requirement, a flexible tube for an endoscope, the flexible tube having a flexible-tube base containing metal as a constituent material and a resin cover layer that covers the outer periphery of the flexible-tube base, in which a primer layer that includes an amino silane coupling agent having a specific structure is provided between the flexible-tube base and the resin cover layer, and the resin cover layer includes a polyurethane elastomer at least on the side in contact with the primer layer.

**SUMMARY OF THE INVENTION**

In addition, in order to smoothly and reliably deliver a tip portion of the flexible tube for an endoscope, the tip portion having a forceps port, an illumination window, etc., to an affected area or the like to obtain detailed information thereof or to perform treatment with high accuracy, it is necessary to enhance the flexibility of the flexible tube for an endoscope to improve the operability. On the other hand, since the endoscope is repeatedly used, the endoscope needs to be washed and disinfected with a chemical each time it is used. Therefore, the flexible tube for an endoscope is also required to have sufficient chemical resistance.

An object of the present invention is to provide a flexible tube for an endoscope, the flexible tube having good elasticity, good flexibility, and sufficient chemical resistance, and an endoscopic medical device that includes the flexible tube for an endoscope. Another object of the present invention is to provide a method for producing the flexible tube for an endoscope and a method for producing the endoscopic medical device.

As a result of extensive studies by the present inventors on a flexible tube for an endoscope, the flexible tube having a primer layer including a silane coupling agent, it has been found that the structure of the flexible-tube base, in particular, the structure of a tubular mesh member is important to further improve the elasticity. The present inventors have found that by using, as a tubular mesh member that forms a flexible-tube base, a mesh member made of braided metal wires and controlling the porosity of the tubular mesh member, the elasticity of the resulting flexible tube can be increased to a high level, and that the flexible tube also has good flexibility and sufficient chemical resistance. Further studies have been conducted on the basis of these findings, and the present invention has been completed.

The above objects of the present invention have been achieved by the following means.

<1> A flexible tube for an endoscope, the flexible tube having a flexible-tube base that is tubular and that has flexibility and a polymer cover layer covering the flexible-tube base,

wherein the flexible-tube base has a spiral tube made of a metal strip and a tubular mesh member covering the spiral tube and made of braided metal wires, the tubular mesh member has a porosity of 2% to 10%, and the flexible tube has, between the flexible-tube base and the polymer cover layer, a primer layer including a silane coupling agent.

<2> The flexible tube for an endoscope according to <1>, wherein the silane coupling agent includes an amino silane coupling agent.

<3> The flexible tube for an endoscope according to <1> or <2>, wherein the polymer cover layer includes a thermoplastic elastomer.

<4> The flexible tube for an endoscope according to any one of <1> to <3>, wherein the polymer cover layer includes, at least on a side in contact with the primer layer, at least one thermoplastic elastomer of a polyurethane elastomer, a polyester elastomer, or a polyamide elastomer.

<5> The flexible tube for an endoscope according to any one of <1> to <4>, wherein metal that constitutes the flexible-tube base is stainless steel.

<6> The flexible tube for an endoscope according to any one of <1> to <5>, wherein metal that constitutes the flexible-tube base has a passivation film on a surface of the metal.

<7> An endoscopic medical device having the flexible tube for an endoscope according to any one of <1> to <6>.

<8> A method for producing the flexible tube for an endoscope according to any one of <1> to <6>, the method including forming a primer layer including a silane coupling agent and a polymer cover layer in this order on an outer periphery of a flexible-tube base containing metal as a constituent material,
wherein the flexible-tube base has a spiral tube made of a metal strip and a tubular mesh member covering the spiral tube and made of braided metal wires, and the tubular mesh member has a porosity of 2% to 10%.

<9> A method for producing an endoscopic medical device, the method including incorporating the flexible tube for an endoscope according to any one of <1> to <6> into an insertion section of an endoscopic medical device.

In the present specification, the term "porosity" refers to a value obtained by the calculation method described in Examples below.

In the description of the present specification, a numerical range represented using "to" means a range that includes a numerical value before "to" as a lower limit and a numerical value after "to" as an upper limit.

The flexible tube for an endoscope according to the present invention as defined in claim 1 has good elasticity, good flexibility, and sufficient chemical resistance.

In the endoscopic medical device according to the present invention, the flexible tube, which is a structural section to be inserted into the body, has good elasticity, good flexibility, and sufficient chemical resistance. Therefore, the endoscopic medical device according to the present invention can further reduce the burden on the subject during use and can repeatedly perform highly accurate medical examinations.

The method for producing a flexible tube for an endoscope according to the present invention as defined in claim 8 can provide a flexible tube for an endoscope, the flexible tube having good elasticity, good flexibility, and sufficient chemical resistance.

According to the method for producing an endoscopic medical device according to the present invention, the flexible tube that forms this device can be made to have good elasticity, good flexibility, and sufficient chemical resistance. Therefore, the method for producing an endoscopic medical device according to the present invention can provide an endoscopic medical device that can further reduce the burden on the subject during use and can repeatedly perform highly accurate medical examinations.

**BRIEF DESCRIPTION OF THE DRAWINGS**

Fig. 1 is an external view illustrating the configuration of an electronic endoscope according to an embodiment;

Fig. 2 is a partial sectional view illustrating the configuration of a flexible tube for an endoscope according to an embodiment;

Fig. 3 is a block diagram illustrating the configuration of an apparatus for producing a flexible tube for an endoscope according to an embodiment;

Fig. 4 is a sectional view taken along line B-B in Fig. 3; and

Fig. 5 is a partial sectional view illustrating the configuration of a tubular mesh member that forms a flexible-tube base according to an embodiment.

**DESCRIPTION OF THE PREFERRED EMBODIMENTS**

Endoscope

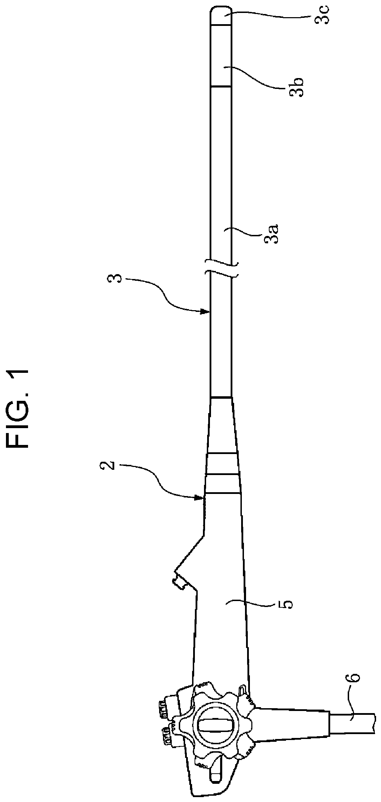

A preferred embodiment of an endoscopic medical device in which a flexible tube for an endoscope according to the present invention is incorporated will be described by taking an electronic endoscope as an example. In the electronic endoscope, a flexible tube for an endoscope (hereinafter, the flexible tube for an endoscope may be simply referred to as a "flexible tube") according to the present invention is incorporated, and the electronic endoscope is used as a medical device for, for example, observing the inside of the body by inserting the flexible tube into the body cavity, the digestive tract, the esophagus, or the like. In an example illustrated in Fig. 1, an electronic endoscope 2 includes an insertion section 3 to be inserted into the body, a main-body operation section 5 connected to a proximal end portion of the insertion section 3, and a universal cord 6 to be connected to a processor device or a light source device. The insertion section 3 is constituted by a flexible tube 3a connected to the main-body operation section 5, an angle portion 3b connected to the flexible tube 3a, and a tip portion 3c connected to the distal end of the angle portion 3b and including therein an imaging device (not illustrated) for capturing an image of the inside of the body. The flexible tube 3a, which accounts for most of the length of the insertion section 3, has flexibility across substantially the entire length thereof and is configured so that, in particular, a portion to be inserted into the inside of the body cavity or the like has higher flexibility.

Flexible Tube for Endoscope

The flexible tube according to the present invention has a flexible-tube base that is tubular and that has flexibility and a polymer cover layer covering the flexible-tube base, and has a primer layer including a silane coupling agent between the flexible-tube base and the polymer cover layer. The flexible-tube base has a spiral tube made of a metal strip and a tubular mesh member covering the spiral tube and made of braided metal wires, and the tubular mesh member has a porosity of 2% to 10%.

The reason why the flexible tube according to the present invention has good elasticity, good flexibility, and sufficient chemical resistance is not clear but is presumed as follows. That is, the tubular mesh member that forms the flexible-tube base and that has a braided structure has a porosity of 2% to 10%, and the primer layer including a silane coupling agent is provided on the flexible-tube base, and thus, in the formation of the polymer cover layer, the inside of pores of the tubular mesh member and the surface of the tubular mesh member (the outer periphery of the spiral tube) are covered with a polymer at a high density. As a result, the reaction area between the silane coupling agent and the polymer cover layer is increased, and the silane coupling agent and the polymer cover layer are effectively bonded to or in close contact with each other. It is considered that one of the reasons why the flexible tube according to the present invention has good elasticity is that the spiral tube and the tubular mesh member are integrally bent when the flexible tube is bent, while the polymer cover layer and the spiral tube are firmly in close contact with each other via the tubular mesh member filled with the polymer at a high density. As demonstrated in Examples described later, it has become clear as an experimental fact that this improvement in the elasticity becomes more apparent when the silane coupling agent has an amino group. The reason for this is also not clear, but presumably, one of the reasons is that when the silane coupling agent has an amino group, the molecular structure of the amino group responsible for bonding or adhesion between the silane coupling agent and the polymer cover layer contributes to an increase in bonding or adhesion points with the polymer cover layer. On the other hand, when the tubular mesh member having a braided structure has a porosity of 2% to 10%, presumably, the contact area between the flexible-tube base and the polymer cover layer can be sufficiently ensured to effectively enhance the adhesiveness, good chemical resistance is also provided, and the above porosity also imparts sufficient flexibility to the flexible-tube base.

Flexible-Tube Base

The flexible tube has, as an innermost layer, a flexible-tube base containing metal as a constituent material.

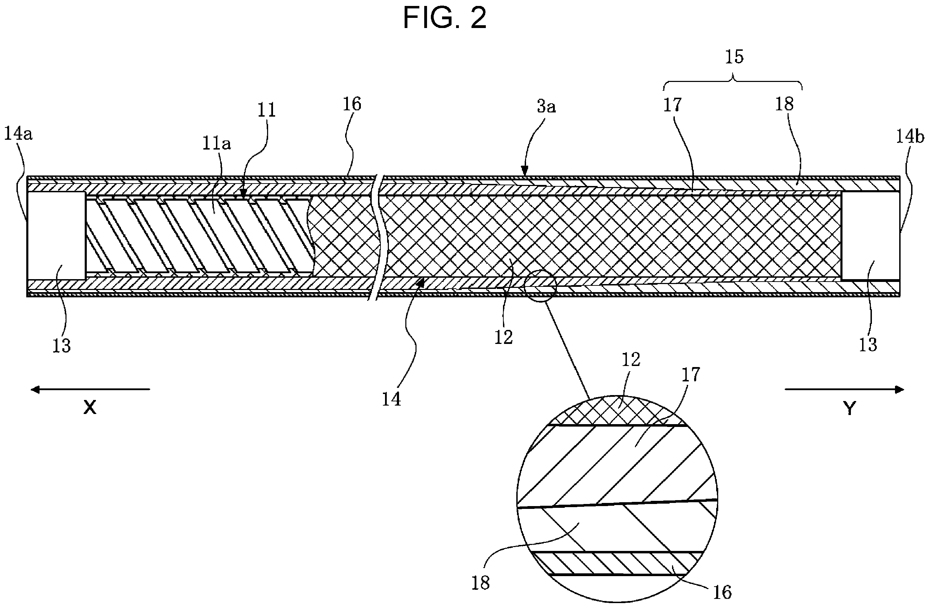

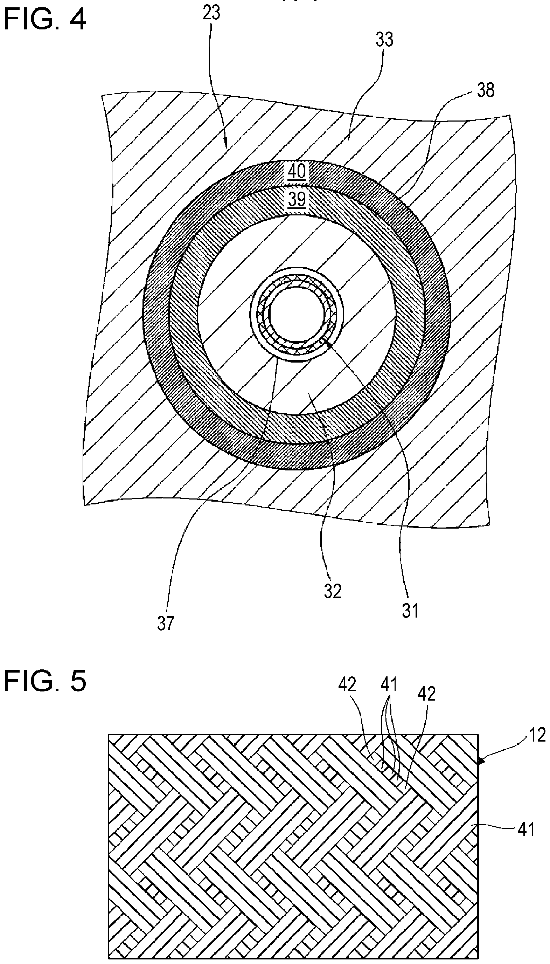

As illustrated in Fig. 2, a flexible-tube base 14 preferably has a form in which a spiral tube 11 disposed on the innermost side and formed by spirally winding a metal strip 11a is covered with a tubular mesh member 12 made of braided metal wires, and caps 13 are fitted to both ends of the flexible-tube base 14. The braided structure of the tubular mesh member 12 is not particularly limited and may be any structure in which metal wires are braided with their upper and lower sides being regularly interchanged. Examples of the braided structure include stockinette (warp/weft), plain weave, twill weave, and satin weave, and, of these, twill weave is preferred. Fig. 5 is a partial sectional view illustrating the configuration of the tubular mesh member 12 having a braided structure formed by twill weave according to an embodiment, in which bundles having a number of ends of three (bundles formed of three metal wires 41) are braided to form pores 42 between the bundles.

The porosity of the tubular mesh member 12 made of braided metal wires is 2% to 10%, and from the viewpoint of elasticity of the flexible tube, the porosity is preferably 3% to 8%, more preferably 3% to 7%, and still more preferably 3% to 6%.

The metal that constitutes the flexible-tube base 14 preferably has a surface that has been subjected to passivation treatment in order to prevent corrosion. That is, the flexible-tube base 14 preferably has a passivation film on an outer periphery (surface) thereof. This passivation treatment can be performed by an ordinary method. A passivation film can be formed on a surface of metal by, for example, immersing the metal in a solution including a strong oxidizing agent such as nitric acid, heating the metal in air (oxygen) or water (water vapor), or anodizing the metal in a solution including an oxidizing agent.

The metal that constitutes the flexible-tube base 14 is preferably stainless steel. The spiral tube 11 formed by spirally winding a metal strip 11a on the innermost side and the tubular mesh member 12 made of braided metal wires are preferably composed of stainless steel. The surface of stainless steel is usually in a state in which chromium and oxygen ($O_2$) are bonded together to form a passivation film. However, even when stainless steel is used as the constituent material of the flexible-tube base 14, the stainless steel is preferably subjected to the passivation treatment described above in order to more reliably form a more uniform passivation film over the entire surface of the stainless steel.

Primer Layer

In the present invention, a primer layer (not illustrated) is disposed on an outer periphery of a flexible-tube base. By providing this primer layer, it is possible to effectively enhance the adhesiveness between the flexible-tube base and a polymer cover layer which will be described later and provided to cover the outer periphery of the flexible-tube base. In the present invention, the primer layer includes a silane coupling agent.

As the silane coupling agent used in the present invention, typical silane coupling agents applicable to a primer layer of a flexible tube for an endoscope can be widely employed. In the present invention, in order to further improve the elasticity, an amino silane coupling agent (preferably a silane coupling agent having at least one of an unsubstituted amino group or a monosubstituted amino group) is preferred. Specific examples of the silane coupling agent include silane coupling agents used in Examples described later, but the present invention is not limited thereto.

The content of the silane coupling agent in the primer layer is preferably 50% by mass or more, more preferably 70% by mass or more, still more preferably 80% by mass or more, and even more preferably 90% by mass or more. The primer layer may be a layer composed of a silane coupling agent.

The content of the amino silane coupling agent in the silane coupling agent is preferably 50% by mass or more, more preferably 70% by mass or more, still more preferably 80% by mass or more, and even more preferably 90% by mass or more. The primer layer may be a layer composed of an amino silane coupling agent.

When the primer layer includes a coupling agent other than the silane coupling agent, the coupling agent is not particularly limited as long as the effects of the present invention are not impaired, and examples thereof include aluminum coupling agents, zirconium coupling agents, and titanium coupling agents.

The thickness of the primer layer is significantly smaller than that of a typical adhesive layer (in other words, the concept of the thickness cannot be conceived). That is, the primer layer that includes a silane coupling agent differs from the adhesive layer that requires a certain thickness and softness for adhesion between the flexible-tube base and the polymer cover layer. Polymer Cover Layer

The flexible tube according to the present invention has a polymer cover layer (a layer including at least one of a resin or an elastomer) on the outer periphery of the flexible-tube base having the primer layer thereon.

In the embodiment in Fig. 2, an outer surface of a polymer cover layer 15 is coated with a topcoat layer 16 that contains fluorine or the like and that contributes to, for example, chemical resistance. In Fig. 2, only a single spiral tube 11 is illustrated, but the spiral tube 11 may be formed by concentrically stacking two or more layers. Note that the polymer cover layer 15 and the topcoat layer 16 in the drawing are drawn to be thicker than the actual thicknesses with respect to the diameter of the flexible-tube base 14 for the sake of clearly illustrating the layer structure.

In the present invention, the polymer cover layer may be formed of a single layer or multiple layers and is preferably formed of multiple layers. This polymer cover layer covers an outer peripheral surface of the flexible-tube base having the primer layer described above. The polymer cover layer 15 in the embodiment in Fig. 2 is not formed of a single layer but has a two-layer structure in which an inner layer 17 that covers the entire peripheral surface around the axis of the flexible-tube base 14 and an outer layer 18 that covers the entire peripheral surface around the axis of the inner layer 17 are laminated. Typically, a soft polymer is used as the material of the inner layer 17 and a hard polymer is used as the material of the outer layer 18, but the present invention is not limited to these embodiments.

When the same polymer cover layer forming material is applied to the flexible-tube base a plurality of times to form a polymer cover layer, this polymer cover layer is formed of a single layer. When different polymer cover layer forming materials are applied to the flexible-tube base a plurality of times to form a polymer cover layer formed of layers containing different types of polymers between adjacent layers, this polymer cover layer is formed of multiple layers.

In the present invention, the polymer cover layer preferably includes a thermoplastic elastomer, and specific examples of the thermoplastic elastomer include fluorine-containing elastomers, polyolefin elastomers, acrylic elastomers, styrene elastomers, polyphenylene oxide elastomers, polyurethane elastomers, polyester elastomers, and polyamide elastomers.

In the present invention, as described later, when the polymer cover layer has a multilayer structure of two or more layers, at least the innermost layer (layer in contact with the primer layer) preferably includes at least one thermoplastic elastomer of a polyurethane elastomer, a polyester elastomer, or a polyamide elastomer, and more preferably includes a polyurethane elastomer. In the present invention, when the polymer cover layer is formed of a single layer, the single-layer polymer cover layer preferably includes at least one thermoplastic elastomer of a polyurethane elastomer, a polyester elastomer, or a polyamide elastomer, and more preferably includes a polyurethane elastomer. That is, in the present invention, the polymer cover layer preferably includes, at least on the side in contact with the primer layer, at least one thermoplastic elastomer of a polyurethane elastomer, a polyester elastomer, or a polyamide elastomer, and more preferably includes a polyurethane elastomer.

Polyurethane Elastomer

As the polyurethane elastomers used in the polymer cover layer, typical polyurethane elastomers applicable to the formation of a flexible tube can be employed. The polyurethane elastomers are each typically obtained by allowing a polyisocyanate, a polyol, and a chain extender to react with each other. The polyurethane elastomer is preferably a random copolymer or block copolymer having a soft segment formed by a reaction between a polymer polyol and a polyisocyanate and a hard segment formed by a reaction between a chain extender and a polyisocyanate, and more preferably a block copolymer.

The polyurethane elastomer is preferably a polyether polyurethane elastomer, a polyester polyurethane elastomer, or a polycarbonate polyurethane elastomer, and more preferably a polyether polyurethane elastomer.

The polyether polyurethane elastomer is preferably an elastomer having a soft segment formed by a reaction between a polyether polyol and a polyisocyanate and the above-described hard segment, that is, a polyurethane elastomer including a polyether in the soft segment. Preferably, this soft segment includes neither an ester bond nor a carbonate bond.

The polyester polyurethane elastomer is preferably an elastomer having a soft segment formed by a reaction between a polyester polyol and a polyisocyanate and the above-described hard segment, that is, a polyurethane elastomer including a polyester in the soft segment. Preferably, this soft segment includes neither an ether bond nor a carbonate bond.

The polycarbonate polyurethane elastomer is preferably an elastomer having a soft segment formed by a reaction between a polycarbonate polyol and a polyisocyanate and the above-described hard segment, that is, a polyurethane elastomer including a polycarbonate in the soft segment. Preferably, this soft segment includes neither an ether bond nor an ester bond.

Examples of the polyisocyanate include diphenylmethane diisocyanate, hexamethylene diisocyanate, tolidine diisocyanate, 1,5-naphthalene diisocyanate, isophorone diisocyanate, and xylylene diisocyanate.

Examples of the polyether polyol include polytetramethylene ether glycol, polydimethylene ether glycol, polytrimethylene ether glycol, polypropylene ether glycol, polyhexamethylene ether glycol, and polyneopentyl ether glycol.

The polyester polyol is obtained by a polycondensation reaction between a dicarboxylic acid and a polyol (preferably a diol). Specific examples of the diol used in the production of polyester polyols include ethylene polyols (e.g., ethanediol), propylene polyols (1,3-propanediol), 1,3-butanediol, 1,4-butanediol, neopentyl polyols (e.g., 1,5-pentanediol), 1,6-hexanediol, and polycaprolactone diol, and these may be used alone or in combination. Examples of the dicarboxylic acid include adipic acid, sebacic acid, and dimer acid, and these may be used alone or in combination.

Examples of the polycaprolactone diol include PLACCEL 205 and PLACCEL 205H (trade names, manufactured by Daicel Corporation), TONE 0200 and TONE 0201 (trade names, manufactured by Union Carbide Corporation), and PLACCEL 208 (trade name, manufactured by Daicel Corporation).

Examples of the polycarbonate polyol include polyethylene carbonate diol, polytetramethylene carbonate diol, and polyhexamethylene carbonate diol.

Examples of the chain extender include aliphatic linear diols having 2 to 6 carbon atoms such as ethanediol, 1,4-butanediol, and 1,6-hexanediol; and 1,4-bis(hydroxyethoxy)benzene. Amines such as hexamethylenediamine, isophoronediamine, tolylenediamine, and monoethanolamine can also be used in combination as needed.

For the polyurethane elastomers according to the embodiment, disclosure of, for example, JP2005-015643A can be referred to. The above polyurethane elastomers may be used alone or in combination of two or more thereof.

Polyester Elastomer

In the present invention, typical polyester elastomers applicable to the formation of a flexible tube can be used as the polyester elastomers.

That is, the polyester elastomers used in the present invention are copolymers having a hard segment composed of a crystalline polyester and a soft segment composed of a polyether or a polyester.

Examples of the hard segment include polybutylene terephthalate and polyethylene terephthalate.

Examples of the soft segment include polyalkylene glycols such as polytetramethylene glycol and polypropylene glycol; bisphenol A-ethylene oxide adducts; bisphenol A-propylene oxide adducts; and polyesters such as polycaprolactone.

In the present invention, preferably, the "polyester elastomers" include neither a urethane bond nor an amide bond in the molecules thereof. The polyester elastomers may be used alone or in combination of two or more thereof.

Polyamide Elastomer

Typical polyamide elastomers applicable to the formation of a flexible tube can be used as the polyamide elastomers. In the present invention, preferably, the polyamide elastomers include neither a urethane bond nor an ester bond. The polyamide elastomers may be used alone or in combination of two or more thereof.

Specific examples of thermoplastic elastomers used in the present invention include the thermoplastic elastomers (fluorine-containing elastomers, polyolefin elastomers, polyurethane elastomers, polyester elastomers, and polyamide elastomers) used in Examples described later, but the present invention is not limited thereto.

The content of the thermoplastic elastomer in the polymer cover layer in the case of a polymer cover layer formed of a single layer and the content of the thermoplastic elastomer in the innermost layer in the case of a polymer cover layer formed of multiple layers are preferably 50% by mass or more, more preferably 70% by mass or more, still more preferably 80% by mass or more, and even more preferably 90% by mass or more. When the polymer cover layer is formed of a single layer, the polymer cover layer may be a layer composed of a thermoplastic elastomer. When the polymer cover layer is formed of multiple layers, the innermost layer may be a layer composed of a thermoplastic elastomer.

A total content of the polyurethane elastomer, the polyester elastomer, and the polyamide elastomer included in the thermoplastic elastomer is preferably 50% by mass or more, more preferably 70% by mass or more, still more preferably 80% by mass or more, and even more preferably 90% by mass or more, and may be 100% by mass.

When the polymer cover layer in the case of a polymer cover layer formed of a single layer and the innermost layer in the case of a polymer cover layer formed of multiple layers include a polymer other than a thermoplastic elastomer, the polymer is not particularly limited as long as the effects of the present invention are not impaired. Examples thereof include vinyl polymers such as acrylic resins and styrene resins, and condensation polymers such as polyesters and polyamides.

When the polymer cover layer is formed of multiple layers, layers other than the innermost layer preferably include at least one thermoplastic elastomer of a polyurethane elastomer, a polyester elastomer, or a polyamide elastomer. An appropriate combination of these thermoplastic elastomers enables the formation of a layer having desired physical properties. When the polymer cover layer is formed of multiple layers, the layers other than the innermost layer more preferably include a polyester elastomer and a polyamide elastomer from the viewpoint of further enhancing elasticity.

In the present invention, a molecular weight of each of the elastomers that can be used in the polymer cover layer is preferably 10,000 to 1,000,000, more preferably 20,000 to 500,000, and particularly preferably 30,000 to 300,000.

In the present invention, the molecular weight of the elastomer refers to a weight-average molecular weight unless otherwise noted. The weight-average molecular weight can be measured by GPC as a molecular weight in terms of polystyrene. In this case, a GPC apparatus HLC-8220 (trade name, manufactured by Tosoh Corporation) is used; an eluant used is chloroform in the case of a polyester elastomer, NMP (N-methyl-2-pyrrolidone) in the case of a polyurethane elastomer, and m-cresol/chloroform (manufactured by Shonan Wako Junyaku K.K.) in the case of a polyamide elastomer; G3000HXL + G2000HXL (each of which is a trade name, manufactured by Tosoh Corporation) are used as columns; the temperature is 23°C; the flow rate is 1 mL/min; and the detection is performed with an RI detector.

As illustrated in Fig. 2, the polymer cover layer 15 in the present invention is preferably formed so as to have a substantially uniform thickness in the longitudinal direction (axial direction) of the flexible-tube base 14. The polymer cover layer 15 has a thickness of, for example, 0.2 mm to 1.0 mm, and the flexible tube 3a has an outer diameter D of, for example, 11 to 14 mm. In Fig. 2, the inner layer 17 and the outer layer 18 are formed such that a ratio of the thickness of each of the layers 17 and 18 to the total thickness of the polymer cover layer 15 changes in the axial direction of the flexible-tube base 14. Specifically, on one end 14a side (distal end side) of the flexible-tube base 14 to be attached to the angle portion 3b, the thickness of the inner layer 17 is larger than the thickness of the outer layer 18 with respect to the total thickness of the polymer cover layer 15. The thickness of the inner layer 17 gradually decreases from the one end 14a toward the other end 14b side (proximal end side) to be attached to the main-body operation section 5. On the other end 14b side, the thickness of the outer layer 18 is larger than the thickness of the inner layer 17.

In Fig. 2, the ratio of the thickness of the inner layer 17 is maximum on the one end 14a, and the ratio of the thickness of the outer layer 18 is maximum on the other end 14b. A ratio of the thickness of the inner layer 17 to the thickness of the outer layer 18 (thickness of inner layer 17:thickness of outer layer 18) may be, for example, 9:1 on the one end 14a, and, for example, 1:9 on the other end 14b. The thicknesses of the two layers are changed such that the ratio of the thickness of the inner layer 17 to the thickness of the outer layer 18 is reversed from the one end 14a to the other end 14b. With this configuration, the flexible tube 3a has a difference in hardness between the one end 14a side and the other end 14b side, and flexibility can be changed in the axial direction such that the one end 14a side is soft and the other end 14b side is hard. The inner layer and the outer layer are preferably formed such that the thickness ratio on the one end is 5:95 to 40:60 (inner layer:outer layer) and the thickness ratio on the other end is 95:5 to 60:40 (inner layer:outer layer).

When the thickness ratio of the inner layer 17 to the outer layer 18 is within the range of 5:95 to 95:5, the amount of extrusion of a polymer that forms a layer having a smaller thickness can also be accurately controlled.

The soft polymer used in the inner layer 17 and the hard polymer used in the outer layer 18 preferably have a difference in 100% modulus, which is an indicator indicating a hardness after molding, of 1 MPa or more and more preferably 3 MPa or more. The difference in melt viscosity, which is an indicator indicating a fluidity of a polymer in a molten state, at a molding temperature of 150°C to 300°C is preferably 2,500 Pa·s or less. With this configuration, the polymer cover layer 15 composed of the inner layer 17 and the outer layer 18 reliably achieves good molding accuracy and a necessary difference in hardness between the distal end side and the proximal end side.

Topcoat Layer

In the flexible tube according to the present invention, the topcoat layer 16 is disposed on an outer periphery of the polymer cover layer 15 as needed. The material of the top coat layer is not particularly limited, but a urethane coating, an acrylic coating, a fluorine coating, a silicone coating, an epoxy coating, a polyester coating, or the like is applied.

Main purposes of use of the topcoat layer are to protect the surface of the flexible tube or make the surface glossy, to impart slidability, and to impart chemical resistance. Therefore, the topcoat layer preferably has a high modulus of elasticity, a smooth surface, and good chemical resistance.

Method for Producing Flexible Tube

A method for producing a flexible tube for an endoscope includes forming a primer layer including a silane coupling agent and a polymer cover layer in this order on an outer periphery of a flexible-tube base containing metal as a constituent material. The method includes forming the primer layer including a silane coupling agent and the polymer cover layer in this order on at least a portion of the outer periphery of the flexible-tube base, and a primer layer including a silane coupling agent and a polymer cover layer may be formed on an inner periphery of the flexible-tube base.

Preparation of Flexible-Tube Base

The flexible-tube base can be prepared by an ordinary method except that a tubular mesh member having a porosity of 2% to 10% is formed by braiding metal wires. For example, referring to JP2007-82802A and JP1997-75299A (JP-H09-75299A), a tubular mesh member having a desired porosity is prepared by controlling, for example, the outer diameters, the number of ends, and the pitch of metal wires, and a spiral tube is covered with this tubular mesh member. Thus, a flexible-tube base can be obtained.

Formation of Primer Layer

In the production of the flexible tube according to the present invention, first, a primer layer is formed on the flexible-tube base. The primer layer can be formed by dissolving a silane coupling agent in a solvent to prepare a coating liquid; forming a coating film on at least an outer periphery of the flexible-tube base by, for example, applying or spraying the coating liquid onto the outer periphery of the flexible-tube base or immersing the flexible-tube base in the coating liquid; and subsequently drying the coating film by an ordinary method (for example, high-temperature drying at about 100°C).

Examples of the solvent that can be used in the coating liquid include alcohol solvents such as methanol and ethanol; ketone solvents such as acetone and methyl ethyl ketone; ester solvents such as ethyl acetate; hydrocarbon solvents such as toluene; and liquid mixtures thereof. It is preferable to further mix water with the solvents in order to accelerate hydrolysis of the silane coupling agent. The coating liquid may be prepared to be acidic (for example, pH 1 to 4 at 25°C) or alkaline (for example, pH 9 to 11 at 25°C).

The content of the silane coupling agent in the coating liquid is not particularly limited, can be, for example, 0.01% by mass to 2% by mass, may be 0.02% by mass or more and less than 0.5% by mass, and may be 0.03% by mass or more and less than 0.4% by mass.

The coating liquid may include, for example, a surfactant and a catalyst besides the silane coupling agent, the solvent, and a pH adjuster. The coating liquid is preferably constituted by the silane coupling agent and the solvent.

In the present invention, a portion that is not covered with the primer layer may be present on the outer periphery of the flexible-tube base as long as the effects of the present invention are not impaired (that is, the primer layer may have a defect in a portion thereof).

Prior to the formation of the primer layer, the flexible-tube base is preferably cleaned by degreasing with an alkali solution, an aqueous solution of a surfactant, an organic solvent, or the like. After the cleaning, the flexible-tube base is preferably further cleaned with water or hot water.

Formation of Polymer Cover Layer

Formation of a polymer cover layer will be described by taking, as an example, a case where the polymer cover layer has a two-layer structure.

A flexible tube that includes a polymer cover layer having a two-layer structure composed of an inner layer and an outer layer can be produced by, for example, melt-kneading and extruding, around the flexible-tube base on which the primer layer has been formed, a first polymer material (for example, a polymer material including a thermoplastic elastomer) that forms the inner layer and a second polymer material that forms the outer layer, thereby covering the flexible-tube base.

In an embodiment in which a polymer cover layer is formed of one layer or three or more layers, the polymer cover layer can also be produced by appropriately changing the layer configuration with reference to the method described below.

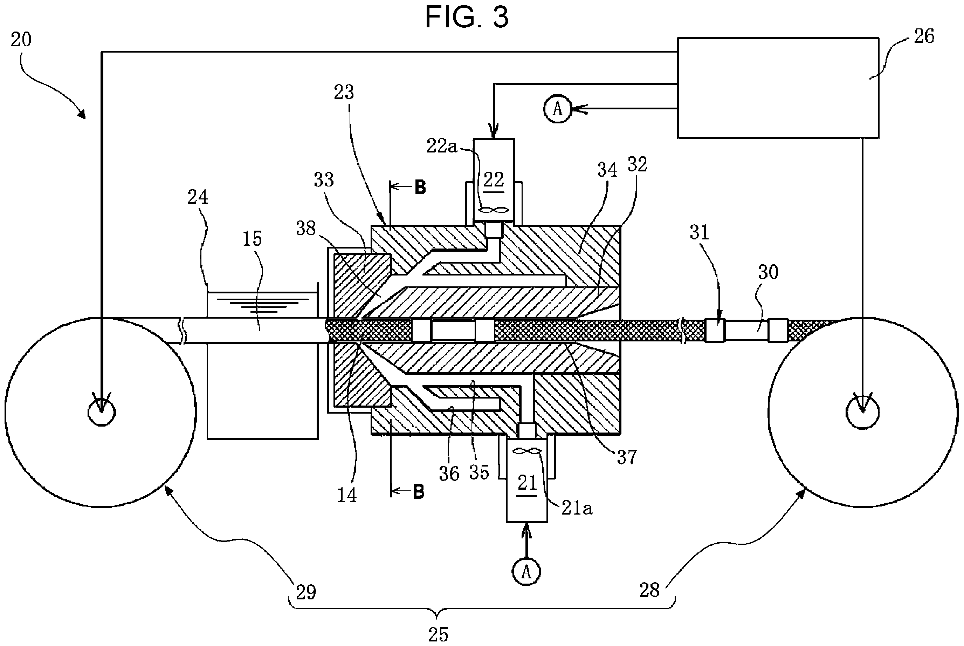

An example of a method for forming a polymer cover layer of the flexible tube 3a (Figs. 1 and 2) will be described with reference to Figs. 3 and 4. In this embodiment, a continuous molding machine is used to mold a polymer cover layer 15. It is preferable to use a continuous molding machine 20 that includes well-known extrusion units 21 and 22 including hoppers, screws 21a and 22a, etc.; a head unit 23 configured to mold a polymer cover layer 15 so as to cover an outer peripheral surface of a flexible-tube base 14; a cooling unit 24; a transport unit 25 (including a feed drum 28 and a take-up drum 29) configured to transport a connected flexible-tube base 31 to the head unit 23; and a control unit 26 configured to control the above units. The head unit 23 preferably includes a nipple 32, a die 33, and a support 34 configured to fixedly support the nipple 32 and the die 33. For example, the apparatus disclosed in Figs. 3 to 5 of JP2011-72391A can be used as an example of the apparatus having the above configuration.

The inside of the die 33 is preferably heated to a predetermined molding temperature. The molding temperature is preferably set in a range of 150°C to 300°C. The temperatures of a first polymer material 39 and a second polymer material 40 can be increased by controlling the temperatures of a heating unit in the apparatus by heating. In addition to this, as the rotational speeds of the screws 21a and 22a become higher, the temperatures of the first polymer material 39 and the second polymer material 40 can be further increased to increase their fluidity. During this process, the molding thicknesses of the inner layer 17 and the outer layer 18 can be adjusted by changing the amounts of the molten first polymer material 39 and second polymer material 40 ejected while the transport speed of the connected flexible-tube base 31 is made constant.

The process of molding the polymer cover layer 15 on the connected flexible-tube base 31 by the continuous molding machine 20 will be described. When the continuous molding machine 20 performs a molding step, the molten first polymer material 39 and second polymer material 40 are respectively extruded from the extrusion units 21 and 22 into the head unit 23. At the same time, the transport unit 25 operates so that the connected flexible-tube base 31 is transported to the head unit 23. During this process, the extrusion units 21 and 22 are in a state of constantly extruding the first polymer material 39 and the second polymer material 40 to feed the polymer materials 39 and 40 to the head unit 23, and the first polymer material 39 and the second polymer material 40 that are respectively extruded from the extrusion units 21 and 22 to gates 35 and 36 pass through an edge and join to each other, and are fed, in a stacked state, through a polymer passage 38 to a molding passage 37. As a result, a two-layer molded polymer cover layer 15 is formed in which an inner layer 17 using the first polymer material 39 and an outer layer 18 using the second polymer material 40 are stacked.

The connected flexible-tube base 31 includes a plurality of flexible-tube bases 14 (each having a primer layer on the outer periphery thereof) that are connected together. While the connected flexible-tube base 31 is transported through the molding passage 37, the polymer cover layer 15 is continuously molded on the plurality of flexible-tube bases 14. When the polymer cover layer 15 is molded from one end 14a side (distal end side) of one flexible-tube base to the other end 14b side (proximal end side) thereof, the thickness of the inner layer 17 is made large immediately after the extrusion units 21 and 22 start the ejection of the polymers. The ratio of the thickness of the outer layer 18 is then gradually increased over an intermediate portion toward the other end 14b side. It is preferable to control the amounts of the polymers ejected in this manner so as to achieve the above-described gradient thickness ratio of the polymer cover layer 15.

Joint members 30 each function as a connecting portion of two flexible-tube bases 14, and thus the control unit 26 is used to switch the amounts of the polymers ejected from the extrusion units 21 and 22. Specifically, the control unit 26 preferably switches the amounts of the polymers ejected from the extrusion units 21 and 22 such that the thickness ratio changes from a thickness ratio on the other end 14b side (proximal end side) of one flexible-tube base 14 to a thickness ratio on one end 14a side (distal end side) of the next flexible-tube base 14. When the polymer cover layer 15 is molded from the one end 14a side of the next flexible-tube base 14 to the other end 14b side thereof, the extrusion units 21 and 22 are preferably similarly controlled such that the thickness of the outer layer gradually increases from the one end side toward the other end side.

The connected flexible-tube base 31 on which the polymer cover layer 15 is molded to the rearmost end is removed from the continuous molding machine 20, and the joint members 30 are then removed from the flexible-tube bases 14 to separate the flexible-tube bases 14 from each other. Next, for each of the separated flexible-tube bases 14, the polymer cover layer 15 is coated with the topcoat layer 16 to complete flexible tubes 3a. The completed flexible tubes 3a are transported to an assembly step of an electronic endoscope.

In the present invention, when the polymer cover layer is formed of multiple layers, a functional layer may be disposed between layers that form the multiple layers.

The above description has been made with reference to the drawings by taking, as an example, an electronic endoscope configured to observe an image of the condition of a subject captured using an imaging device; however, the present invention is not limited thereto and is also applicable to an endoscope configured to observe the condition of a subject by employing an optical image guide.

Endoscopic Medical Device and Method for Producing the Same

The flexible tube according to the present invention is widely applicable to endoscopic medical devices. For example, the flexible tube according to the present invention is applicable to an endoscope equipped with a clip or wire at the distal end thereof or to a device equipped with a basket or brush. Note that the term "endoscopic medical device" is meant to broadly include medical devices or diagnosis and treatment devices that include an insertion section having flexibility and that are introduced and used in the body, such as remote-controlled medical devices, in addition to medical devices including an endoscope as a basic structure, as described above.

An endoscopic medical device according to the present invention has an insertion section in which the flexible tube for an endoscope according to the present invention is incorporated. That is, a method for producing an endoscopic medical device according to the present invention includes incorporating the flexible tube for an endoscope according to the present invention into an insertion section of an endoscopic medical device.

Examples

Hereinafter, the present invention will be described in more detail by way of Examples; however, these Examples should not be construed as limiting the present invention. Preparation of Coating Liquid for Forming Primer Layer

A solution having a ratio water/ethanol of 5/75 on a mass basis was prepared. A coupling agent shown in Table 1 below was dissolved in the solution so as to have a concentration of 8.9 g/kg, and the resulting solution was used as a coating liquid for forming a primer layer.

Preparation of Coating Liquid for Forming Adhesive Layer

In 1 kg of methyl ethyl ketone, 100 g of a polyester polyurethane (trade name: N-2304, manufactured by Nippon Polyurethane Industry Co., Ltd.) and 10 g of a polyisocyanate (trade name: CORONATE, manufactured by Nippon Polyurethane Industry Co., Ltd.) were dissolved, and the resulting solution was used as a coating liquid for forming an adhesive layer.

Preparation of Flexible-Tube for Endoscope

A flexible tube having the structure illustrated in Fig. 2 was prepared. The polymer cover layer had a single-layer structure or a two-layer structure as shown in Table 1 below. Preparation of Flexible-Tube Base

A flexible-tube base having a form in which a spiral tube 11 was formed using a metal strip 11a made of stainless steel, and the spiral tube 11 was covered with a tubular mesh member 12 made of braided stainless steel fibers (metal wires) was prepared. The spiral tube 11 used was a tube in which the width of the metal strip 11a and the gap between the strips were controlled so as to have a bending radius of 12.5 (mm). The tubular mesh member 12 having the porosity shown in Table 1 below was obtained by setting the element wire diameter (outer diameter of each of the metal wires 41) of the tubular mesh member 12 to 0.1 mm and setting the core diameter to 10.5 mm, and controlling the number of ends, the number of carriers, and the pitch in braiding (twill weaving) with a braiding machine. The outer periphery of the spiral tube 11 was covered with the tubular mesh member 12 obtained as described above to prepare a flexible-tube base.

The flexible-tube base has a length of 80 cm and a diameter of 12 mm. This stainless steel flexible tube has a passivation layer on a surface thereof, the passivation layer being formed by annealing treatment (heating treatment) in the formation of the spiral tube 11 and the tubular mesh member 12.

Method for Calculating Porosity

Five 10 mm-square sheets were cut out at random from the tubular mesh member. A central region (a 5 mm $\times$ 5 mm region centered on the intersection of two diagonal lines) of each of the sheets was observed with a digital microscope (VHX-200 (trade name), manufactured by Keyence Corporation) to determine the area of pores (the area of opening portions of pores) X $mm^2$. A porosity A (%) of each sheet was determined from the following formula, and the number-average value thereof (the value (%) determined by dividing the total of the porosities A by 5) was defined as the porosity specified in the present invention.

$$\text{Porosity A} = 100 \times \text{X mm}^2/25 \text{ mm}^2$$

Formation of Primer Layer (Examples 1 to 50 and Comparative Examples 1 to 30)
The flexible-tube base was cleaned by immersing in a 7.5% aqueous sodium hydroxide solution at 60°C for one minute. Subsequently, the flexible-tube base was rinsed with distilled water and then dried in an oven at 100°C for 10 minutes. The cleaned flexible-tube base was immersed in the above-prepared coating liquid for forming a primer layer at room temperature for one minute and then dried in an oven at 160°C for 10 minutes. Thus, a flexible-tube base having a primer layer on the outer periphery (coating surface) was prepared.

Formation of Adhesive Layer (Comparative Example 31)

The above-prepared solution for forming an adhesive layer was uniformly applied to the outer periphery of the stainless steel flexible-tube base and dried at room temperature for two hours. Subsequently, heat treatment was further performed at 150°C for two hours to prepare a flexible-tube base having an adhesive layer on the outer periphery (resin-coated surface). The adhesive layer had a thickness of about 80 $\mu$m.

Formation of Polymer Cover Layer

The outer periphery of the flexible-tube base having the primer layer or the adhesive layer was covered with an elastomer as shown in Table 1 below by extrusion (molding temperature: 200°C) to prepare a flexible tube for an endoscope, the flexible tube having a polymer cover layer. The polymer cover layer had a thickness of 0.4 mm (in the case of a two-layer structure, the total thickness of the two layers was 0.4 mm).
In the cases where the polymer cover layer is formed of two layers (Examples 1 to 39 and 44 to 46 and Comparative Examples 1 to 31), the two layers were simultaneously formed to cover the outer periphery by two-layer extrusion molding. In these cases, the inner/outer layer ratios at the distal end and at the proximal end were inner layer:outer layer = 80:20 at the distal end and inner layer: outer layer = 20:80 at the proximal end, respectively.

[Test Example 1] Evaluation of Flexibility

The flexible tube for an endoscope prepared as described above was bent at a position 40 cm from one tip portion, and the radius of curvature when the flexible tube was not bent was measured and evaluated on the basis of the evaluation criteria described below. "C" or higher is satisfactory.

Evaluation of Flexibility

A: The radius of curvature is less than 15 mm.
B: The radius of curvature is 15 mm or more and less than 17.5 mm.
C: The radius of curvature is 17.5 mm or more and less than 20 mm.
D: The radius of curvature is 20 mm or more.

The results are shown in the tables below.

[Test Example 2] Evaluation of Elasticity

In an environment at a temperature of 25°C and a relative humidity of 50%, positions 30 cm and 50 cm from one tip portion of the above-prepared flexible tube for an endoscope were fixed, and a position of 40 cm (central portion of the flexible tube) was pushed in 15 mm in a direction (diameter direction) perpendicular to the length direction of the flexible tube. A ratio of a repulsive force (b) after 30 seconds to a repulsive force (a) after 0.1 seconds was measured as an elasticity (%). The repulsive force was measured with a force gauge (ZTS50N, manufactured by IMADA CO., LTD.).

$$[\text{Elasticity (\%)}] = [(b)/(a)] \times 100$$

The elasticity was evaluated on the basis of the evaluation criteria described below. "C" or higher is satisfactory.

Evaluation Criteria for Elasticity

AA: The elasticity is 80% or more.
A: The elasticity is 75% or more and less than 80%.
B: The elasticity is 70% or more and less than 75%.
C: The elasticity is 60% or more and less than 70%.
D: The elasticity is less than 60%.

[Test Example 3] Evaluation of Peracetic Acid Resistance of Flexible Tube

The flexible tube for an endoscope prepared as described above was sealed such that liquid did not enter the inside, and then immersed in a 7.0% aqueous peracetic acid solution at 55°C for 150 hours. Subsequently, the surface was sufficiently washed with water. After washing with water, drying was performed at 23°C × 50% RH (relative humidity) for 24 hours. After drying, for the polymer cover layer of the flexible tube for an endoscope, a cut having a width of 1 cm and a length of 10 cm was formed in the axial direction of the flexible tube such that the cut reached the flexible-tube base. The flexible-tube base and the polymer cover layer were peeled from each other at a constant rate of 2 mm/min in the axial direction along the cut with a width of 1 cm to measure a 90° peel strength (this value is referred to as measured value X). The peel strength was measured with a force gauge (unit: N/cm). Each flexible tube for an endoscope, the flexible tube not having been immersed in the aqueous peracetic acid solution, was used to measure the 90° peel strength in the same manner (measured value Y). For all the flexible tubes, the 90° peel strength was measured under the same conditions.
A ratio (%, 100 × X/Y) of the measured value X to the measured value Y was determined and evaluated on the basis of the following criteria. "C" or higher is satisfactory.

Evaluation Criteria for Peracetic Acid Resistance

A: The ratio is 80% or more.

B: The ratio is 60% or more and less than 80%.

C: The ratio is 40% or more and less than 60%.

D: The ratio is less than 40% (a flexible tube whose polymer cover layer was peeled off during immersion in the aqueous peracetic acid solution is included).

Table 1

| Table 1-1 | | | | | | | | | | |
|---|---|---|---|---|---|---|---|---|---|---|
| | Comparative Example 1 | Example 1 | Example 2 | Example 3 | Example 4 | Example 5 | Example 6 | Example 7 | Comparative Example 2 | Comparative Example 3 |
| Porosity [%] | 1 | 2 | 3 | 5 | 6 | 7 | 9 | 10 | 11 | 12 |
| Polymer cover layer | Outer PE1<br><br>Inner PU1 | Outer PE1<br>Inner PU1 | Outer PE1<br>Inner PU1 | Outer PE1<br>Inner PU1 | Outer PE1<br>Inner PU1 | Outer PE1<br>Inner PU1 | Outer PE1<br>Inner PU1 | Outer PE1<br>Inner PU1 | Outer PE1<br><br>Inner PU1 | Outer PE1<br><br>Inner PU1 |
| Primer layer | (S-1) | (S-1) | (S-1) | (S-1) | (S-1) | (S-1) | (S-1) | (S-1) | (S-1) | (S-1) |
| Flexibility | D | C | A | A | A | A | A | A | A | A |
| Elasticity | B | A | AA | AA | AA | A | A | B | D | D |
| Peracetic acid resistance | A | A | A | A | A | A | A | A | A | A |

14

EP 4 224 236 B1

Table 1-2

|  | Example 8 | Example 9 | Example 10 | Example 11 | Example 51 | Example 52 |
|---|---|---|---|---|---|---|
| Porosity [%] | 3 | 3 | 3 | 3 | 3 | 3 |
| Polymer cover layer | Outer PE1 Inner PU1 | Outer PE1 Inner PU1 | Outer PE1 Inner PU1 | Outer PE1 Inner PU1 | Outer PA1 Inner PE1 | Outer PE1 Inner PA1 |
| Primer layer | (S-2) | (S-3) | (S-4) | (S-5) | (S-1) | (S-1) |
| Flexibility | A | A | A | A | A | A |
| Elasticity | AA | AA | AA | AA | A | A |
| Peracetic acid resistance | A | A | A | A | A | A |

Table 1-3

| | Comparative Example 4 | Example 12 | Example 13 | Example 14 | Example 15 | Example 16 | Example 17 | Example 18 | Comparative Example 5 | Comparative Example 6 |
|---|---|---|---|---|---|---|---|---|---|---|
| Porosity [%] | 1 | 2 | 3 | 5 | 6 | 7 | 9 | 10 | 11 | 12 |
| Polymer cover layer | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 |
| Primer layer | (S-6) | (S-6) | (S-6) | (S-6) | (S-6) | (S-6) | (S-6) | (S-6) | (S-6) | (S-6) |
| Flexibility | D | C | A | A | A | A | A | A | A | A |
| Elasticity | B | B | B | B | B | B | C | C | D | D |
| Peracetic acid resistance | A | A | A | A | A | A | A | A | A | A |

Table 2

| Table 1-4 | | | | | | | | | | |
|---|---|---|---|---|---|---|---|---|---|---|
| | Comparative Example 7 | Example 19 | Example 20 | Example 21 | Example 22 | Example 23 | Example 24 | Example 25 | Comparative Example 8 | Comparative Example 9 |
| Porosity [%] | 1 | 2 | 3 | 5 | 6 | 7 | 9 | 10 | 11 | 12 |
| Polymer cover layer | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 |
| Primer layer | (S-7) | (S-7) | (S-7) | (S-7) | (S-7) | (S-7) | (S-7) | (S-7) | (S-7) | (S-7) |
| Flexibility | D | C | A | A | A | A | A | A | A | A |
| Elasticity | B | B | B | B | B | B | C | c | D | D |
| Peracetic acid resistance | A | A | A | A | A | A | A | A | A | A |

Table 1-5

| | Comparative Example 10 | Example 26 | Example 27 | Example 28 | Example 29 | Example 30 | Example 31 | Example 32 | Comparative Example 11 | Comparative Example 12 |
|---|---|---|---|---|---|---|---|---|---|---|
| Porosity [%] | 1 | 2 | 3 | 5 | 6 | 7 | 9 | 10 | 11 | 12 |
| Polymer cover layer | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 |
| Primer layer | (S-8) | (S-8) | (S-8) | (S-8) | (S-8) | (S-8) | (S-8) | (S-8) | (S-8) | (S-8) |
| Flexibility | D | C | A | A | A | A | A | A | A | A |
| Elasticity | B | B | B | B | B | B | C | C | D | D |
| Peracetic acid resistance | A | A | A | A | A | A | A | A | A | A |

Table 1-6

| | Comparative Example 13 | Example 33 | Example 34 | Example 35 | Example 36 | Example 37 | Example 38 | Example 39 | Comparative Example 14 | Comparative Example 15 |
|---|---|---|---|---|---|---|---|---|---|---|
| Porosity [%] | 1 | 2 | 3 | 5 | 6 | 7 | 9 | 10 | 11 | 12 |
| Polymer cover layer | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 |
| Primer layer | (S-9) | (S-9) | (S-9) | (S-9) | (S-9) | (S-9) | (S-9) | (S-9) | (S-9) | (S-9) |
| Flexibility | D | C | A | A | A | A | A | A | A | A |
| Elasticity | B | B | B | B | B | B | C | C | D | D |
| Peracetic acid resistance | A | A | A | A | A | A | A | A | A | A |

Table 3

| Table 1-7 | | | | | | | | | | |
|---|---|---|---|---|---|---|---|---|---|---|
| | Comparative Example 16 | Comparative Example 17 | Comparative Example 18 | Comparative Example 19 | Comparative Example 20 | Comparative Example 21 | Comparative Example 22 | Comparative Example 23 | Comparative Example 24 | Comparative Example 25 |
| Porosity [%] | 3 | 3 | 3 | 3 | 3 | 3 | 3 | 3 | 3 | 3 |
| Polymer cover layer | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 | Outer PE1 Inner PU1 |
| Primer layer | (A-1) | (A-2) | (A-3) | (A-4) | (A-5) | (Z-1) | (Z-2) | (Z-3) | (Z-4) | (Z-5) |
| Flexibility | A | A | A | A | A | A | A | A | A | A |
| Elasticity | D | D | D | D | D | D | D | D | D | D |
| Peracetic acid resistance | A | A | A | A | A | A | A | A | A | A |

Table 1-8

| | Comparative Example 26 | Comparative Example 27 | Comparative Example 28 | Comparative Example 29 | Comparative Example 30 |
|---|---|---|---|---|---|
| Porosity [%] | 3 | 3 | 3 | 3 | 3 |
| Polymer cover layer | Outer PE1 Inner PU1 | Outer PE1 Inner PU1 | Outer PE1 Inner PU1 | Outer PE1 Inner PU1 | Outer PE1 Inner PU1 |
| Primer layer | (T-1) | (T-2) | (T-3) | (T-4) | (T-5) |
| Flexibility | A | A | A | A | A |
| Elasticity | D | D | D | D | D |
| Peracetic acid resistance | A | A | A | A | A |

Table 1-9

| | Example 40 | Example 41 | Example 42 | Example 43 | Example 44 | Example 45 | Example 46 | Example 47 | Example 48 | Comparative Example 31 |
|---|---|---|---|---|---|---|---|---|---|---|
| Porosity [%] | 3 | 3 | 3 | 3 | 3 | 3 | 3 | 3 | 3 | 3 |
| Polymer cover layer | PU1 | PU2 | PU3 | PU4 | Outer PE1 Inner PU2 | Outer PA1 Inner PU1 | Outer PA1 Inner PU2 | Fluorine-containing elastomer | Olefin elastomer | Outer PE1 Inner PU1 |
| Primer layer | (S-1) | (S-1) | (S-1) | (S-1) | (S-1) | (S-1) | (S-1) | (S-1) | (S-1) | Adhesive |
| Flexibility | A | A | A | A | A | A | A | A | A | A |
| Elasticity | A | A | B | B | AA | AA | AA | C | C | D |
| Peracetic acid resistance | A | A | A | A | A | A | A | A | A | D |

Table 1-10

|  | Example 49 | Example 50 |
|---|---|---|
| Porosity [%] | 3 | 3 |
| Polymer cover layer | PE1 | PA1 |
| Primer layer | (S-1) | (S-1) |
| Flexibility | A | A |
| Elasticity | B | B |
| Peracetic acid resistance | A | A |

Note in Tables

The polymers used in the polymer cover layer are as follows.

PU1:
Polyether polyurethane elastomer (trade name: PANDEX T-8185, manufactured by DIC Corporation)
PU2:
Polyether polyurethane elastomer (trade name: Miractran E380, manufactured by Nippon Polyurethane Industry Co., Ltd.)
PU3:
Polyester polyurethane elastomer (trade name: Miractran E480, manufactured by Nippon Polyurethane Industry Co., Ltd.)
PU4:
Polycarbonate polyurethane elastomer (trade name: PANDEX T-9280, manufactured by DIC Corporation)
PE1:
Polyester elastomer (trade name: PELPRENE P-40B, manufactured by Toyobo Co., Ltd.)
PA1:
Polyamide elastomer (trade name: PEBAX 4533, manufactured by Arkema Inc.)

Fluorine-containing elastomer: DAI-EL T-530 (trade name), manufactured by Daikin Industries, Ltd.

Polyolefin elastomer: Zelas MC707 (trade name), manufactured by Mitsubishi Chemical Corporation

In Examples 1 to 39 and 44 to 46 and Comparative Examples 1 to 31, "inner" means the innermost layer of the polymer cover layer, and "outer" means the outermost layer of the polymer cover layer.

The coupling agents used in the primer layer are as follows.

Silane Coupling Agents

(S-1):
N-Methylaminopropyltrimethoxysilane, manufactured by Tokyo Chemical Industry Co., Ltd.
(S-2):
3-Aminopropyltrimethoxysilane (trade name: KBM-903, manufactured by Shin-Etsu Chemical Co., Ltd.)
(S-3):
3-Ureidopropyltrialkoxysilane (trade name: KBE-585, manufactured by Shin-Etsu Chemical Co., Ltd.)
(S-4):
N-2-(Aminoethyl)-3-aminopropylmethyldimethoxysilane (trade name: KBM-603, manufactured by Shin-Etsu Chemical Co., Ltd.)
(S-5):
N-Phenyl-3-aminopropyltrimethoxysilane (trade name: KBM-573, manufactured by Shin-Etsu Chemical Co., Ltd.)
(S-6):
3-Trimethoxysilylpropylsuccinic anhydride (trade name: X-12-967C, manufactured by Shin-Etsu Chemical Co., Ltd.)

(S-7):
(3-Methacryloxypropyl)trimethoxysilane (trade name: KBM-503, manufactured by Shin-Etsu Chemical Co., Ltd.)
(S-8):
3-Glycidoxypropyltrimethoxysilane (trade name: KBM-403, manufactured by Shin-Etsu Chemical Co., Ltd.)
(S-9):
3-Mercaptopropyltrimethoxysilane (trade name: KBM-803, manufactured by Shin-Etsu Chemical Co., Ltd.)

Aluminum Coupling Agents

(A-1):
Aluminum sec-butoxide (trade name: ASBD, manufactured by Kawaken Fine Chemicals Co., Ltd.)
(A-2) :
Aluminum trisacetylacetonate (trade name: ORGATIX AL-3100, manufactured by Matsumoto Fine Chemical Co., Ltd.)
(A-3):
Aluminum bisethylacetoacetate monoacetylacetonate (trade name: ORGATIX AL-3200, manufactured by Matsumoto Fine Chemical Co., Ltd.)
(A-4) :
Aluminum trisethylacetoacetate (trade name: ORGATIX AL-3215, manufactured by Matsumoto Fine Chemical Co., Ltd.)
(A-5):
Aluminum octadecylacetoacetate diisopropylate (trade name: PLENACT AL-M, manufactured by Ajinomoto Fine-Techno Co., Inc.)

Zirconium Coupling Agents

(Z-1):

Zirconium tetra-n-propoxide (trade name: ORGATIX ZA-45, manufactured by Matsumoto Fine Chemical Co., Ltd.)
(Z-2):
Zirconium tetra-n-butoxide (trade name: ORGATIX ZA-65, manufactured by Matsumoto Fine Chemical Co., Ltd.)
(Z-3):
Zirconium tetraacetylacetonate (trade name: ORGATIX ZC-150, manufactured by Matsumoto Fine Chemical Co., Ltd.)
(Z-4):
Zirconium lactate ammonium salt (trade name: ORGATIX ZC-300, manufactured by Matsumoto Fine Chemical Co., Ltd.)
(Z-5):
Zirconium tri-n-butoxide stearate (trade name: ORGATIX ZC-320, manufactured by Matsumoto Fine Chemical Co., Ltd.)

Titanium Coupling Agents

(T-1):
Tetra-n-butyl titanate (trade name: ORGATIX TA-21, manufactured by Matsumoto Fine Chemical Co., Ltd.)
(T-2):
n-Butyl titanate dimer (trade name: ORGATIX TA-23, manufactured by Matsumoto Fine Chemical Co., Ltd.)
(T-3):
Isopropyl triisostearoyl titanate (trade name: PLENACT TTS, manufactured by Ajinomoto Fine-Techno Co., Inc.)
(T-4):
Dioctyl bis(ditridecyl)phosphate titanate (trade name: PLENACT 46B, manufactured by Ajinomoto Fine-Techno Co., Inc.)

(T-5):
Diisopropyl bis(dioctyl pyrophosphate) titanate (trade name: PLENACT 38S, manufactured by Ajinomoto Fine-Techno Co., Inc.)

The flexible tubes of Comparative Examples 1, 4, 7, 10, and 13, which had a porosity lower than the lower limit specified in the present invention, had poor flexibility. The flexible tubes of Comparative Examples 2, 3, 5, 6, 8, 9, 11, 12, 14, and 15, which had a porosity exceeding the upper limit specified in the present invention, had poor elasticity. The flexible tubes of Comparative Examples 16 to 30 each have a primer layer composed of a coupling agent other than a silane coupling agent. The flexible tubes of Comparative Examples 16 to 30 had poor elasticity, although the flexible tubes had a porosity satisfying the range specified in the present invention. The flexible tube of Comparative Example 31, which had an adhesive layer instead of the primer layer, had poor elasticity and chemical resistance.

In contrast, it is found that the flexible tubes according to the present invention (Examples 1 to 50) each have good elasticity, good flexibility, and sufficient chemical resistance. The comparison between the results of Examples 1 to 11 and the results of Examples 12 to 39 shows that when an amino silane coupling agent is used as the silane coupling agent, the elasticity can be increased to a higher level by controlling the porosity.

Reference Signs List

2 electronic endoscope (endoscope)
3 insertion section
3a flexible tube
3b angle portion
3c tip portion

5 main-body operation section
6 universal cord
11 spiral tube
11a metal strip

12 tubular mesh member
13 cap
14 flexible-tube base
14a distal end side
14b proximal end side

15 polymer cover layer
16 topcoat layer
17 inner layer
18 outer layer
X angle portion 3b side (soft)
Y main-body operation section 5 side (hard)
20 continuous molding machine (production apparatus)
21, 22 extrusion unit
21a screw
22a screw

23 head unit
24 cooling unit
25 transport unit
26 control unit
28 feed drum
29 take-up drum
30 joint member
31 connected flexible-tube base
32 nipple
33 die

| 34 | support |
| 35, 36 | gate |
| 37 | molding passage |
| 38 | polymer passage |
| 39 | soft polymer |
| 40 | hard polymer |
| 41 | metal wire |
| 42 | pore |

**Claims**

1.  A flexible tube for an endoscope, the flexible tube comprising:

    a flexible-tube base that is tubular and that has flexibility; and
    a polymer cover layer covering the flexible-tube base,
    wherein the flexible-tube base has a spiral tube made of a metal strip and a tubular mesh member covering the spiral tube and made of braided metal wires,
    and
    the flexible tube comprises, between the flexible-tube base and the polymer cover layer, a primer layer including a silane coupling agent, **characterized in that** the tubular mesh member has a porosity of 2% to 10%.

2.  The flexible tube for an endoscope according to claim 1, wherein the silane coupling agent includes an amino silane coupling agent.

3.  The flexible tube for an endoscope according to claim 1 or 2, wherein the polymer cover layer includes a thermoplastic elastomer.

4.  The flexible tube for an endoscope according to any one of claims 1 to 3, wherein the polymer cover layer includes, at least on a side in contact with the primer layer, at least one thermoplastic elastomer of a polyurethane elastomer, a polyester elastomer, or a polyamide elastomer.

5.  The flexible tube for an endoscope according to any one of claims 1 to 4, wherein metal that constitutes the flexible-tube base is stainless steel.

6.  The flexible tube for an endoscope according to any one of claims 1 to 5, wherein metal that constitutes the flexible-tube base has a passivation film on a surface of the metal.

7.  An endoscopic medical device comprising the flexible tube for an endoscope according to any one of claims 1 to 6.

8.  A method for producing the flexible tube for an endoscope according to any one of claims 1 to 6, the method comprising:

    forming a primer layer including a silane coupling agent and a polymer cover layer in this order on an outer periphery of a flexible-tube base containing metal as a constituent material,
    wherein the flexible-tube base has a spiral tube made of a metal strip and a tubular mesh member covering the spiral tube and made of braided metal wires, **characterized in that**
    the tubular mesh member has a porosity of 2% to 10%.

9.  A method for producing an endoscopic medical device, the method comprising:
    incorporating the flexible tube for an endoscope according to any one of claims 1 to 6 into an insertion section of an endoscopic medical device.

**Patentansprüche**

1.  Flexibler Schlauch für ein Endoskop, wobei der flexible Schlauch Folgendes umfasst:

eine Basis des flexiblen Schlauchs, die schlauchförmig ist und die Flexibilität aufweist; und
eine Polymerdeckschicht, welche die Basis des flexiblen Schlauchs bedeckt,
wobei die Basis des flexiblen Schlauchs einen Spiralschlauch, der aus einem Metallstreifen gefertigt ist, und ein schlauchförmiges Maschenelement, das den Spiralschlauch bedeckt und aus geflochtenen Metalldrähten gefertigt ist, aufweist, und
der flexible Schlauch zwischen der Basis des flexiblen Schlauchs und der Polymerdeckschicht eine Grundierungsschicht umfasst, die ein Silankopplungsmittel beinhaltet,
**dadurch gekennzeichnet, dass** das schlauchförmige Maschenelement eine Porosität von 2 % bis 10 % aufweist.

2. Flexibler Schlauch für ein Endoskop nach Anspruch 1, wobei das Silankopplungsmittel ein Aminosilankopplungsmittel beinhaltet.

3. Flexibler Schlauch für ein Endoskop nach Anspruch 1 oder 2, wobei die Polymerdeckschicht ein thermoplastisches Elastomer beinhaltet.

4. Flexibler Schlauch für ein Endoskop nach einem der Ansprüche 1 bis 3, wobei die Polymerdeckschicht zumindest auf einer Seite in Kontakt mit der Grundierungsschicht, zumindest ein thermoplastisches Elastomer aus einem Polyurethanelastomer, einem Polyesterelastomer oder einem Polyamidelastomer beinhaltet.

5. Flexibler Schlauch für ein Endoskop nach einem der Ansprüche 1 bis 4, wobei Metall, das die Basis des flexiblen Schlauchs darstellt, Edelstahl ist.

6. Flexibler Schlauch für ein Endoskop nach einem der Ansprüche 1 bis 5, wobei Metall, das die Basis des flexiblen Schlauchs darstellt, einen Passivierungsfilm auf einer Oberfläche des Metalls aufweist.

7. Endoskopische medizinische Vorrichtung, die den flexiblen Schlauch für ein Endoskop nach einem der Ansprüche 1 bis 6 umfasst.

8. Verfahren zum Herstellen des flexiblen Schlauchs für ein Endoskop nach einem der Ansprüche 1 bis 6, wobei das Verfahren Folgendes umfasst:

Bilden einer Grundierungsschicht beinhaltend ein Silankopplungsmittel und einer Polymerdeckschicht in dieser Reihenfolge auf einem Außenumfang einer Basis des flexiblen Schlauchs, die Metall als Bestandsmaterial enthält,
wobei die Basis des flexiblen Schlauchs einen Spiralschlauch, der aus einem Metallstreifen gefertigt ist, und ein schlauchförmiges Maschenelement, das den Spiralschlauch bedeckt und aus geflochtenen Metalldrähten gefertigt ist, aufweist,
**dadurch gekennzeichnet, dass** das schlauchförmige Maschenelement eine Porosität von 2 % bis 10 % aufweist.

9. Verfahren zum Herstellen einer endoskopischen medizinischen Vorrichtung, wobei das Verfahren Folgendes umfasst:
Einbauen des flexiblen Schlauchs für ein Endoskop nach einem der Ansprüche 1 bis 6 in einen Einführabschnitt einer endoskopischen medizinischen Vorrichtung.

**Revendications**

1. Tube flexible pour un endoscope, le tube flexible comprenant :

une base de tube flexible qui est tubulaire et qui comporte de la flexibilité ; et
une couche de revêtement de polymère recouvrant la base du tube flexible,
ladite base du tube flexible comportant un tube en spirale constitué d'une bande métallique et d'un élément de maille tubulaire recouvrant le tube en spirale et constitué de fils métalliques tressés, et
ledit tube flexible comprenant, entre la base du tube flexible et la couche de revêtement de polymère, une couche d'apprêt comprenant un agent de couplage de silane,
**caractérisé en ce que** l'élément de maille tubulaire comporte une porosité de 2 % à 10 %.

**2.** Tube flexible pour un endoscope selon la revendication 1, ledit agent de couplage de silane comprenant un agent de couplage de silane aminé.

**3.** Tube flexible pour un endoscope selon la revendication 1 ou 2, ladite couche de revêtement de polymère comprenant un élastomère thermoplastique.

**4.** Tube flexible pour un endoscope selon l'une quelconque des revendications 1 à 3, ladite couche de revêtement de polymère comprenant, au moins sur un côté en contact avec la couche d'apprêt, au moins un élastomère thermoplastique d'un élastomère de polyuréthane, d'un élastomère de polyester ou d'un élastomère de polyamide.

**5.** Tube flexible pour un endoscope selon l'une quelconque des revendications 1 à 4, ledit métal qui constitue la base du tube flexible étant de l'acier inoxydable.

**6.** Tube flexible pour un endoscope selon l'une quelconque des revendications 1 à 5, ledit métal qui constitue la base du tube flexible comportant un film de passivation sur une surface du métal.

**7.** Dispositif médical endoscopique comprenant le tube flexible pour un endoscope selon l'une quelconque des revendications 1 à 6.

**8.** Procédé de production du tube flexible pour un endoscope selon l'une quelconque des revendications 1 à 6, le procédé comprenant :

la formation d'une couche d'apprêt comprenant un agent de couplage de silane et une couche de revêtement de polymère dans cet ordre sur la périphérie extérieure d'une base de tube flexible contenant du métal en tant que matériau constitutif,
ladite base du tube flexible comportant un tube en spirale constitué d'une bande métallique et d'un élément de maille tubulaire recouvrant le tube en spirale et constitué de fils métalliques tressés,
**caractérisé en ce que** l'élément de maille tubulaire comporte une porosité de 2 % à 10 %.

**9.** Procédé de production d'un dispositif médical endoscopique, le procédé comprenant :
l'incorporation du tube flexible pour un endoscope selon l'une quelconque des revendications 1 à 6 dans une section d'insertion d'un dispositif médical endoscopique.

EP 4 224 236 B1

# FIG. 1

# FIG. 2

EP 4 224 236 B1

# FIG. 3

EP 4 224 236 B1

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019013243 A
- JP 2005015643 A
- JP 2007082802 A
- JP 9075299 A
- JP H0975299 A
- JP 2011072391 A